# EUROPEAN PATENT SPECIFICATION

(11) **EP 4 186 504 B1**
(45) Date of publication and mention of the grant of the patent: **29.05.2024**
(21) Application number: 21210499.6
(22) Date of filing: 25.11.2021
(51) Int. Cl.: A61K 31/4045, A61K 9/08, A61K 47/10, A61K 47/26, A61K 47/22, A61K 47/46, A61P 25/20

(54) **ORAL PHARMACEUTICAL AQUEOUS SOLUTIONS COMPRISING MELATONIN AND USE THEREOF**
ORALE PHARMAZEUTISCHE WÄSSRIGE LÖSUNGEN MIT MELATONIN UND DEREN VERWENDUNG
SOLUTIONS AQUEUSES PHARMACEUTIQUES ORALES COMPRENANT DE LA MÉLATONINE ET LEUR UTILISATION

(43) Date of publication of application: 31.05.2023
(73) Proprietor: Alissa Healthcare Research Limited, Whiteley, Hampshire PO15 7FE (GB)
(72) Inventor: DAVIES, Robin, Whiteley, Hampshire - PO15 7FE (GB)
(74) Representative: Bianchetti & Minoja with Trevisan & Cuonzo IPS SRL

(56) References cited:
- EP-A1- 3 530 289
- WO-A1-2019/038586
- WO-A1-2019/161470
- CN-A- 105 687 186

## Description

### TECHNICAL FIELD

The present invention relates to oral pharmaceutical aqueous solutions comprising melatonin.

Furthermore, the invention relates to oral pharmaceutical aqueous solutions comprising melatonin for use in the prevention and/or treatment of insomnia or sleep disorders.

### BACKGROUND OF THE INVENTION

Melatonin, with chemical name *N*-acetyl-5-methoxy-tryptamine or *N*-(2-(5-methoxy-1*H*-indol-3-yl)ethyl)acetamide, having the following structural formula: 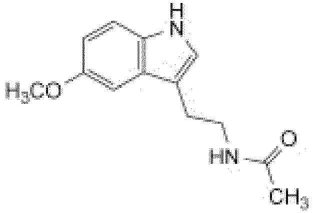 is a substance found in animals, plants, fungi, and bacteria. In animals, it is a hormone that anticipates the daily onset of darkness, however in other organisms, it may have different functions. Likewise, the synthesis of melatonin in animals differs from that in the other organisms.

In animals, melatonin is involved in the entrainment (synchronization) of the circadian rhythms of physiological functions including sleep timing, blood pressure regulation, seasonal reproduction and many others. Many of melatonin's biological effects in animals are produced through activation of melatonin receptors, while others are due to its role as a pervasive and powerful antioxidant, with a particular role in the protection of nuclear and mitochondrial DNA.

The activity of melatonin at the MT1, MT2 and MT3 receptors is believed to contribute to its sleep-promoting properties, as these receptors (mainly MT1 and MT2) are involved in the regulation of circadian rhythms and sleep regulation.

Because of the role of melatonin in sleep and circadian rhythm regulation, and the age-related decrease in endogenous melatonin production, melatonin may effectively improve sleep quality.

Several formulations of melatonin are widely available worldwide.

EP 3 206 665 B1 is directed to a substantially water-free parenteral bulk solution consisting of an anhydrous liquid preparation of melatonin containing at least:
a) melatonin (MLT) quantitatively at the concentration of or higher than 10.0% weight/volume of the bulk solution;
b) a polyethoxylated derivative (PED) selected among macrogolglycerol hydroxystearate, preferably polyoxyl 40 hydrogenated castor oil, or macrogolglycerol ricinoleate, preferably polyoxyl 35 castor oil, or macrogol 15 hydroxystearate, also polyoxyl 15 hydroxystearate, or a mixture thereof;
   whereas the mass ratio MLT/PED is 1: 1 (weight/weight); and
c) ethanol 10 volumes.

WO2013/068565 relates to a powder obtained by spray drying comprising melatonin, leucine as a soluble excipient and a water-soluble surfactant. The pharmaceutical composition is obtained by dissolving the powder in water and 5-40% of a polyalkylene glycol, preferably polyethylene glycol, to obtain a melatonin concentration of 3-30% by weight.

WO2015144965 is directed to parenteral formulations of melatonin comprising polyethylene glycol and polypropylene glycol.

WO 2016058985 discloses concentrated melatonin solution, wherein melatonin is present in a quantity of 10.0% or higher in a substantially water-free carrier mixture of ethanol and a polyethoxylated derivative. The concentrated solution, free of preserving agents, is suitable to prepare injectable sterile compositions for parenteral administration, or formulations for topical or oral administration.

WO2021038601 discloses an aqueous composition of melatonin comprising melatonin or melatonin in any acceptable form and at least one ingredient selected from a group consisting of solubilizers, crystal inhibitors, stabilizers, ad-oxidants, solubilizer which is also an anti-oxidant, chelating agents, complexing agents and a combination thereof wherein the concentration of melatonin in the composition is 0.5 mg/ml to 10 mg/ml and the aqueous composition is essentially free of preservative and optionally free of surfactant and co-solvent.

EP 3 530 289 A1 is directed to oral pharmaceutical solution comprising melatonin, propylene glycol within the range from 20 mg/ml to 500 mg/ml, sorbitol within the range from 20 mg/ml to 400 mg/ml and purified water, wherein the pH of the oral solution is from 3.0 to 5.0 and wherein the oral solution is free of parabens, glycerol, sorbic acid, sodium or potassium benzoate. WO 2019/038586 A1 discloses oral pharmaceutical aqueous solutions comprising melatonin, an anti-oxidant and a sweetening agent.

There is still the need to develop pharmaceutical formulation comprising melatonin, which are stable and pleasant-tasting at the same time.

### SUMMARY OF THE INVENTION

The present invention is directed to oral pharmaceutical aqueous solutions comprising melatonin, from 1 to 10 mg/ml of benzyl alcohol, propylene glycol, an anti-oxidant, a sweetening agent and a flavor.

### DETAILED DISCLOSURE OF THE INVENTION

The scope of the present invention is to provide a stable, aqueous pharmaceutical solution for oral administration comprising melatonin characterized by a pleasant-tasting.

It has been surprisingly found that oral pharmaceutical aqueous solutions comprising melatonin, from 1 to 10 mg/ml of benzyl alcohol, propylene glycol, an anti-oxidant agent, a sweetening agent and optionally a flavor are stable and pleasant-tasting.

Relatively high concentration of benzyl alcohol, necessary to achieve antimicrobial resistance of formulations, are known to give strong burning of the mouth, causing bad taste which is a drawback in formulations for oral administration.

Thanks to the aqueous pharmaceutical solutions of the invention, it is possible to achieve chemical stability of melatonin and good physical and microbiological stability of the formulation in the aqueous vehicle along with a pleasant taste, which increases patients' compliance with treatment. Furthermore, the risk of contamination by opportunistic microbial pathogens, resulting in potential health consequences, is prevented in the solution of the invention.

The amount of melatonin in the aqueous pharmaceutical solution may vary from 0.5 to 3 mg/ml of aqueous solution, preferably the amount of melatonin is 1 mg/ml.

The melatonin content over time is equal to or not less than 95.0% by weight on its initial total amount, a melatonin content equal to or higher than 95.0% by weight on its initial total amount is retained in the formulation stored for 3-months at +40 °C (LC method, Ph. Eur. 2.2.29).

According to a preferred embodiment the content of related impurity substances of melatonin is equal or lower than 0.1% by weight for 5-Methoxytryptamine (5-TMT), equal or lower than 0.4% for any other secondary impurity, equal or lower than 1.0% by weight for sum of impurity.

The amount of benzyl alcohol in the aqueous pharmaceutical solution may vary from 1 to 10 mg/ml of aqueous solution, preferably the amount of benzyl alcohol is 6 mg/ml.

The benzyl alcohol content over time is equal to or not less than 90.0% by weight on its initial total amount, a benzyl alcohol content equal to or higher than 90.0% by weight on its initial total amount is retained in the formulation stored for 3-months at +40 °C (LC method, Ph. Eur. 2.2.29).

The anti-oxidant agent is preferably sodium ascorbate in a concentration from 0.1 to 5 mg/ml of aqueous solution, preferably 1 mg/ml.

The amount of propylene glycol may vary from 30 to 100 mg/ml of aqueous solution, preferably 50-55 mg/ml.

The sweetening agent is preferably sucralose.

The amount of sweetening agent in the solution may range from 0.1 to 1 mg/ml of aqueous solution, preferably 0.5 mg/ml.

The oral pharmaceutical aqueous solutions of the invention may optionally comprise a flavor for flavouring and further improving the organoleptic properties of oral solutions.

The flavor is preferably strawberry flavor as strawberry flavour 113034 with specific odour of strawberry in liquid form.

The amount of flavor in the solution is preferably 0.0001 ml/ml.

The oral pharmaceutical aqueous solution may also comprise L-Arg, reduced glutathione or L-Met, preferably L-Arg.

The aqueous vehicle contains water, optionally in combination with a co-solvent.

Water is used is used as solvent, preferably it is purified water, water for the preparation of medicines other than those that are required to be both sterile and apyrogenic (Ph. Eur.).

According to a preferred embodiment the oral pharmaceutical aqueous has the following composition:

**Table 1**

| **Component** | **Amount/1 ml of formulation** | **Amount/5 ml dose of solution** |
|---|---|---|
| Melatonin | 1 mg | 5 mg |
| Sucralose | 0.5 mg | 2.5 mg |
| Benzyl alcohol | 6 mg | 30 mg |
| Sodium ascorbate | 1 mg | 5 mg |
| Propylene glycol | 52 mg | 260 mg |
| Strawberry flavour | 0.0001 ml | 0.0005 ml |
| Water | qs to 1 ml | qs to 5 ml |

The pharmaceutical aqueous solution of the invention may be prepared by conventional methods.

The pharmaceutical aqueous solution is prepared by dissolving the formulation comprising melatonin, benzyl alcohol, propylene glycol, an anti-oxidant agent, a sweetening agent and optionally a flavor into an aqueous vehicle.

According to a preferred embodiment, the oral pharmaceutical aqueous solution are characterized by a microbiological contamination wherein:
- total aerobic microbial count (TAMC): not more than 10² per 1 ml and total combined yeasts and molds count (TYMC): not more than 10¹ per 1 ml (Ph. Eur. 2.6.12, 2.6.13);
- absence of Escherichia coli in 1 mL (Ph. Eur. 5.1.4).

The present invention is also directed to the use of oral pharmaceutical aqueous solutions in the prevention and/or treatment of insomnia, for example as a short-term treatment of primary insomnia characterised by poor quality of sleep in patients, and sleep disorders as jet lag disorder (circadian rhythm sleep-wake disorder). Furthermore, the invention relates to a method for treating insomnia and sleep disorders comprising administering to a patient in need thereof oral pharmaceutical aqueous solution comprising melatonin according to the present invention.

The following examples further illustrate the invention.

### EXAMPLES

### Example 1 - Oral pharmaceutical aqueous solution containing melatonin

A melatonin 1 mg/ml formulation in form of oral aqueous solution has been prepared. The composition of the solution is reported in Table 2 below.

The so obtained formulation is a slightly yellow to yellow transparent solution, with strawberry odour.

**Table 2**

| No | Names of ingredients | Unit formula | Function | References to standards |
|---|---|---|---|---|
| | | In 1 ml | | |
| 1 | Melatonin | 1.00 mg | Active ingredient | BP |
| 2 | Sucralose | 0.50 mg | Sweetener | Ph. Eur. * |
| 3 | Benzyl alcohol | 6.20 mg | Solvent and preservative | Ph. Eur. * |
| 4 | Sodium ascorbate | 1.00 mg | Antioxidant | Ph. Eur. * |
| 5 | Propylene glycol (1,2-propanediol) | 52.00 mg | Solvent and preservative | Ph. Eur. * |
| 6 | Strawberry flavour 113034 | 0.0001 mg | Flavour | In-house |
| 7 | Water, purified | q.s. to 1 ml | Solvent | Ph. Eur. * |
| | Total: | 1.00 ml | | * current edition |

### Example 2 - Evaluation of melatonin stability in the solution

Stability of melatonin in the pharmaceutical solution of Example 1 was evaluated using stressed storage conditions, i.e. keeping the solution in climatic chambers at +40⁰C/75% RH for testing weekly encompassing 1-month period, and further monthly covering 3 months in total. Main parameters studied were visual observation on formulation colour changes over time, melatonin damage detected by RP-HPLC analysis method and microbial contamination. Visual test showed that after 4 weeks of storage the formulation changed colour to slightly yellow.

RP-HPLC results of melatonin analysis allowed quantifying of area percent of the principal melatonin peak, the indication on the retention of its integrity and possible damage/degradation of the molecule.

The respective data are summarized in Table 3 showing that more than 96% of melatonin remained in formulations.

**Table 3**

| Melatonin maintenance (area percent of principal peak, RP-HPLC analysis) in formulations of Example 1 containing different concentration of benzyl alcohol kept at stress conditions (+40°C) for 3 months | | |
|---|---|---|
| **Area percent for the principal peak, %** | | |
| **Concentration of benzyl alcohol mg/ml** | **Initial time analysis** | **After 3 months storage** |
| 0.6 | 97.29 | 96.84 |
| 0.9 | 96.61 | 95.14 |

### Example 3 - Evaluation of efficacy on antimicrobial preservation

Testing of antimicrobial efficiency of solutions with benzyl alcohol concentrations varied from 0.6 mg/ml to 0.9 mg/ml were made using control micro-organisms to assess if European Pharmacopeia requirements were met.

The method used for preservative effectiveness evaluation is as described in European Pharmacopoeia monograph 5.1.3. Requirements for oral preparations are described therein: after inoculation of pure culture of specified control micro-organism into drug product the titre of the bacteria must be reduced by 3-folds in 14 days (log reduction must be not less than 3), and no further increase of viable micro-organisms is allowed for next two weeks. For moulds and yeasts the quantity of micro-organisms must drop 10 times until 14th day of incubation, and no increase is allowed until next testing date (after 28 days incubation).

Data showed that the benzyl alcohol concentration is sufficient for inhibition of microbial growth of *Aspergillus niger*, *Candida albicans*, *Escherichia coli*, *Pseudomonas aeruginosa* and *Staphylococcus aureus* in melatonin solutions.

For oral preparations recommended criteria are:

**Table 4**

| **Log₁₀ reduction** | | |
|---|---|---|
| | **14d** | **28d** |
| Bacteria | 3 | ≥1 |
| Fungi | 1 | ≥1 |

### Example 4 - Evaluation of antimicrobial preservative efficacy in the solution of Example 1

The microbiological quality of the solution of Example 1 (melatonin 1 mg/ml oral solution) has been tested according to the requirements of Ph. Eur. 5.1.4 Microbiological quality of non-sterile pharmaceutical preparations and substances for pharmaceutical use for aqueous preparations for oral use. Standardized indicators of microbiological quality are:
TAMC, (CFU/g or CFU/mL) - < 10²
TYMC, (CFU/g or CFU/mL) - < 10¹
Escherichia coli (1 g or 1 ml) - Absence

### Antimicrobial Preservative Testing

Antimicrobial Preservative Testing of melatonin 1 mg/ ml oral solution have been tested according to Ph. Eur. 5.1.3.

Efficacy of antimicrobial preservation of the product meets the requirements described in the current edition of the European Pharmacopeia monograph 5.1.3.

### Results

Antimicrobial preservative efficacy has been observed and results are presented below in Tables 5, 6 and 7.

**Table 5**

| log₁₀ reduction in number of viable micro-organisms at 14^{th} day | | | | | |
|---|---|---|---|---|---|
| Test organism | Avg. Titre (0), (CFU/mL) | Avg. Titre (14 days),(CFU/mL) | log₁₀ red(14 days) | Requirements | Conforms |
| *S. aureus* | 5.48 × 10⁵ | 1.00 × 10¹ | >4.3 | ≥ 3 | Yes |
| *P. aeruginosa* | 1.19 × 10⁶ | 1.00 × 10¹ | >5.1 | ≥ 3 | Yes |
| *E. coli* | 5.15 × 10⁵ | 1.00 × 10¹ | >4.7 | ≥ 3 | Yes |
| *C. albicans* | 0.73 × 10⁵ | 1.00 × 10¹ | >3.9 | ≥ 1 | Yes |
| *A. brasiliensis* | 4.2 × 10⁵ | 2.00 × 10¹ | >5.3 | ≥ 1 | Yes |

**Table 6**

| log₁₀ reduction in number of viable micro-organisms at 28^{th} day | | | | | |
|---|---|---|---|---|---|
| Test organism | Avg. Titre (14 days),(CFU/mL) | Avg. Titre (28 days),(CFU/mL) | logic red (28 days) | Requirements | Conforms |
| *S. aureus* | 1.00 × 10¹ | 1.00 × 10¹ | NI | NI | Yes |
| *P. aeruginosa* | 1.00 × 10¹ | 1.00 × 10¹ | NI | NI | Yes |
| *E. coli* | 1.00 × 10¹ | 1.00 × 10¹ | NI | NI | Yes |
| *C. albicans* | 1.00 × 10¹ | 1.00 × 10¹ | NI | NI | Yes |
| *A. brasiliensis* | 2.00 × 10¹ | 1.00 × 10¹ | >0.3 | NI | Yes |

### Conclusion

Acceptance criteria evaluation

**Table 7**

| **Test suitability** | | |
|---|---|---|
| **Requirements** | **Results** | **Conforms** |
| No evident growth occurs in all negative control samples | No Growth | Yes |
| Initial *S*. *aureus* titter in the drug product must be between 10⁵ - 10⁶ CFU/mL* | 4.06 × 10⁵ | Yes |
| Initial *P. aeruginosa* titter in the drug product must be between 10⁵ - 10⁶ CFU/mL* | 3.98 × 10⁵ | Yes |
| Initial *E. coli* titter in the drug product must be between 10⁵ - 10⁶ CFU/mL* | 3.96 × 10⁵ | Yes |
| Initial *C*. *albicans* titter in the drug product must be between 10⁵ - 10⁶ CFU/mL* | 0.62 × 10⁵ | Yes |
| Initial *A. brasiliensis* titter in the drug product must be between 10⁵ - 10⁶ CFU/mL* | 4.02 × 10⁵ | Yes |

| Analysis results | | |
|---|---|---|
| **Acceptance criteria** | **Results** | **Conform** |
| For bacteria log₁₀ reduction in number of viable micro-organisms after 14d must be not less than 3 | >4.7 | Yes |
| For bacteria no increase in number of viable micro-organisms compared to previous reading after 28 d incubation period must be obtained | No Increase | Yes |
| For fungi log₁₀ reduction in number of viable micro-organisms after 14d must be not less than 1 | >3.9 | Yes |
| For fungi no increase in number of viable micro-organisms compared to previous reading after 28 d incubation period must be obtained | No Increase | Yes |

| | | |
|---|---|---|
| * - *minimum acceptable count* = *0.5×10⁵ CFU, and maximum acceptable count* = *2×10⁶ CFU* | | |

## Claims

1. Oral pharmaceutical aqueous solutions comprising melatonin, from 1 to 10.0 mg/ml of benzyl alcohol, propylene glycol, an anti-oxidant and a sweetening agent.

2. Oral pharmaceutical aqueous solutions according to claim 1 further comprising a flavor.

3. Oral pharmaceutical aqueous solutions according to claim 1 or 2, wherein the concentration of melatonin is in the range of 0.5-3 mg/ml.

4. Oral pharmaceutical aqueous solutions according to claim 3, wherein the concentration of melatonin is 1 mg/ml.

5. Oral pharmaceutical aqueous solutions according to any one of claims from 1 to 4, wherein the sweetening agent is sucralose.

6. Oral pharmaceutical aqueous solutions according to any one of claims from 1 to 5, wherein the anti-oxidant is sodium ascorbate.

7. Oral pharmaceutical aqueous solutions according to any one of claims from 1 to 6, wherein the concentration of propylene glycol is in the range of 20 to 100 mg/ml.

8. Oral pharmaceutical aqueous solutions according to claim 7, wherein the concentration of propylene glycol is of 52 mg/ml..

9. Oral pharmaceutical aqueous solutions according to any one of claims from 1 to 8 having the following composition:
| **Component** | **Amount/1 ml of formulation** | **Amount/5 ml dose of solution** |
|---|---|---|
| Melatonin | 1 mg | 5 mg |
| Sucralose | 0.5 mg | 2.5 mg |
| Benzyl alcohol | 6 mg | 30 mg |
| Sodium ascorbate | 1 mg | 5 mg |
| Propylene glycol | 52 mg | 260 mg |
| Strawberry flavour | 0.0001 ml | 0.0005 ml |
| Water | qs to 1 ml | qs to 5 ml |

10. Oral pharmaceutical aqueous solutions according to any one of claims from 1 to 9 for use as a medicament.

11. Oral pharmaceutical aqueous solutions according to any one of claims from 1 to 9 for use in the prevention and/or treatment of insomnia, sleep disorders, jet lag disorders.

## Patentansprüche

1. Orale pharmazeutische wässrige Lösungen, umfassend Melatonin, 1 bis 10,0 mg/ml Benzylalkohol, Propylenglycol, ein Antioxidans und ein Süßungsmittel.

2. Orale pharmazeutische wässrige Lösungen nach Anspruch 1, weiter umfassend ein Aroma.

3. Orale pharmazeutische wässrige Lösungen nach Anspruch 1 oder 2, wobei die Konzentration an Melatonin im Bereich von 0,5-3 mg/ml liegt.

4. Orale pharmazeutische wässrige Lösungen nach Anspruch 3, wobei die Konzentration an Melatonin 1 mg/ml beträgt.

5. Orale pharmazeutische wässrige Lösungen nach einem der Ansprüche 1 bis 4, wobei das Süßungsmittel Sucralose ist.

6. Orale pharmazeutische wässrige Lösungen nach einem der Ansprüche 1 bis 5, wobei das Antioxidans Natriumascorbat ist.

7. Orale pharmazeutische wässrige Lösungen nach einem der Ansprüche 1 bis 6, wobei die Konzentration an Propylenglycol im Bereich von 20 bis 100 mg/ml liegt.

8. Orale pharmazeutische wässrige Lösungen nach Anspruch 7, wobei die Konzentration an Propylenglycol 52 mg/ml beträgt.

9. Orale pharmazeutische wässrige Lösungen nach einem der Ansprüche 1 bis 8 mit der folgenden Zusammensetzung:
| **Komponente** | **Menge/1 ml Formulierung** | **Menge/5 ml-Dosis Formulierung** |
|---|---|---|
| Melatonin | 1 mg | 5 mg |
| Sucralose | 0,5 mg | 2,5 mg |
| Benzylalkohol | 6 mg | 30 mg |
| Natriumascorbat | 1 mg | 5 mg |
| Propylenglycol | 52 mg | 260 mg |
| Erdbeeraroma | 0,0001 ml | 0,0005 ml |
| Wasser | qs bis 1 ml | qs bis 5 ml |

10. Orale pharmazeutische wässrige Lösungen nach einem der Ansprüche 1 bis 9 zur Verwendung als ein Medikament.

11. Orale pharmazeutische wässrige Lösungen nach einem der Ansprüche 1 bis 9 zur Verwendung bei der Prävention und/oder Behandlung von Schlaflosigkeit, Schlafstörungen, Jetlag-Störungen.

## Revendications

1. Solutions aqueuses pharmaceutiques orales comprenant de la mélatonine, de 1 à 10,0 mg/ml d'alcool benzylique, du propylène glycol, un antioxydant et un agent édulcorant.

2. Solutions aqueuses pharmaceutiques orales selon la revendication 1 comprenant en outre un arôme.

3. Solutions aqueuses pharmaceutiques orales selon la revendication 1 ou 2, dans lesquelles la concentration en mélatonine est comprise dans la plage de 0,5 à 3 mg/ml.

4. Solutions aqueuses pharmaceutiques orales selon la revendication 3, dans lesquelles la concentration en mélatonine est de 1 mg/ml.

5. Solutions aqueuses pharmaceutiques orales selon l'une quelconque des revendications 1 à 4, dans lesquelles l'agent édulcorant est le sucralose.

6. Solutions aqueuses pharmaceutiques orales selon l'une quelconque des revendications 1 à 5, dans lesquelles l'antioxydant est l'ascorbate de sodium.

7. Solutions aqueuses pharmaceutiques orales selon l'une quelconque des revendications 1 à 6, dans lesquelles la concentration en propylène glycol est comprise dans la plage de 20 à 100 mg/ml.

8. Solutions aqueuses pharmaceutiques orales selon la revendication 7, dans lesquelles la concentration en propylène glycol est de 52 mg/ml.

9. Solutions aqueuses pharmaceutiques orales selon l'une quelconque des revendications 1 à 8 ayant la composition suivante :
| **Constituant** | **Quantité/1 ml de formulation** | **Quantité/dose de 5 ml de solution** |
|---|---|---|
| Mélatonine | 1 mg | 5 mg |
| Sucralose | 0,5 mg | 2,5 mg |
| Alcool benzylique | 6 mg | 30 mg |
| Ascorbate de sodium | 1 mg | 5 mg |
| Propylène glycol | 52 mg | 260 mg |
| Arôme fraise | 0,0001 ml | 0,0005 ml |
| Eau | qs pour 1 ml | qs pour 5 ml |

10. Solutions aqueuses pharmaceutiques orales selon l'une quelconque des revendications 1 à 9 pour une utilisation en tant que médicament.

11. Solutions aqueuses pharmaceutiques orales selon l'une quelconque des revendications 1 à 9 pour une utilisation dans la prévention et/ou le traitement de l'insomnie, des troubles du sommeil, des troubles du décalage horaire.
